# EUROPEAN PATENT APPLICATION

(11) **EP 4 793 964 A1**
(43) Date of publication of application: **19.08.2026**
(21) Application number: 25157332.5
(22) Date of filing: 12.02.2025
(51) Int. Cl.: G16H 30/40, A61M 37/00

(54) **SYSTEM, DEVICE AND METHOD FOR MEDICAL IMAGING**

(71) Applicant: Imec VZW, 3001 Leuven (BE)
(72) Inventor: Gross, Patrick, Crawley Down, RH10 4HD (GB)
(74) Representative: Roth, Sebastian

(57) **Abstract**

An imaging system (100) for medical imaging is provided. The imaging system comprises at least one training device (101), and at least one imaging device (102) operably coupled to the at least one training device (101), wherein the at least one imaging device (102) being configured to be attached to the skin of a person at a predefined location. In this regard, the at least one imaging device (102), in combination with the at least one training device (101), is configured to generate a first set of images corresponding to the predefined location, whereby the at least one training device (101) is configured to generate training data corresponding to the predefined location based on the first set of images. Furthermore, the at least one imaging device (102) is further configured to exclusively generate a second set of images corresponding to the predefined location based on the training data.

## Description

The invention relates to medical imaging and/or targeted drug delivery, particularly to medical therapeutic imaging and/or targeted therapeutic drug delivery, in a largely autonomous manner.

Generally, when delivering drugs intravascularly, e.g., either intravenously or via a catheter, at least a significant proportion circulates systemically, potentially causing toxicity. In addition, depending on the molecular size and biochemical properties of the drugs, they may find it difficult to cross from the vasculature into the target tissue, e.g., crossing the blood-brain-barrier.

Both these problems can be addressed using bubble agents that are caused to oscillate at their resonant frequency using ultrasound. Such oscillations can open and make the vasculature more permeable at and/or release drugs by bursting bubbles containing them. This can be done in a locally targeted manner using ultrasound.

For example, the document US 2024/0131366 Al discloses a Low-Intensity Non-Focused Ultrasound (LINFU) method and device for creating a wide area of stable cavitation through resonance of intravenously introduced microbubbles inside the entire pancreas or another organ sized area of the body.

However, in situations where there is a benefit for a targeted delivery regime to be applied over an extended period of time, using existing technology, such as the LINFU method of US 2024/0131366 A1, especially using non-trainable devices, e.g., based on operator or trained professionals, may not be feasible. Additionally, the automation of said heavy and rigid devices is not practical, as it means the user of such devices, e.g., a patient, is likely to have to remain stationary for a long time and any motion may degrade the acoustic coupling.

Accordingly, an object of the invention is to provide a system, a device, and a method for medical therapeutic imaging and/or targeted drug delivery in a reliable autonomous manner.

The object is solved by the features of the first independent claim for the system, by the features of the second independent claim for the device, and by the features of the third independent claim for the method. The dependent claims contain further developments.

According to a first aspect of the invention, an imaging system for medical imaging is provided. The imaging system comprises at least one training device, and at least one imaging device operably coupled to the at least one training device, wherein the at least one imaging device being configured to be attached to the skin of a person at a predefined location.

In this regard, the at least one imaging device, in combination with the at least one training device, is configured to generate a first set of images corresponding to the predefined location, whereby the at least one training device is configured to generate training data corresponding to the predefined location based on the first set of images.

Furthermore, the at least one imaging device is further configured to exclusively generate a second set of images corresponding to the predefined location based on the training data.

The term imaging can be understood as acoustic imaging performed by the at least one imaging device at the predefined location, e.g., corresponding to a target organ. Additionally or alternatively, the term imaging can be understood as spatial and/or temporal sensing using sensing data, either unreconstructed/raw sensing data or processed sensing data, generated by the at least one imaging device.

In this regard, the at least one imaging device, in combination with the at least one training device, may generate a first set of sensing data, e.g., from spatial and/or temporal sensing at the predefined location, e.g., corresponding to a target organ corresponding to the predefined location. The at least one training device may thereby generate training data corresponding to the predefined location based on the first set of sensing data.

Afterwards, the at least one imaging device may exclusively generate, preferably from a remote location corresponding to the at least one training device, a second set of sensing data corresponding to the predefined location based on the training data. Advantageously, the relative location of a particular anatomy can be deduced and targeted drug delivery, especially using bubble agents, can be performed in a largely autonomous manner.

Preferably, the at least one training device comprises an ultrasound imaging device or an x-ray imaging device or a computed tomography, CT, device or a magnetic resonance imaging, MRI, device or a positron emission tomography, PET, device or a combination thereof.

Preferably, the first set of images and the second set of images are ultrasound images, e.g., Doppler ultrasound imaging and/or sensing, brightness mode (B-Mode) imaging and/or sensing, harmonic imaging and/or sensing, and so on.

Preferably, the training data comprises instructions for the operating frequency of the at least one imaging device and/or focus of the second set of images corresponding to the predefined location and/or depth of the second set of images corresponding to the predefined location and/or attachment anomalies between the at least one imaging device and the skin. Advantageously, the reliability can be further improved.

Preferably, the training data further comprises parameters for focused ultrasound based on the first set of images and the at least one imaging device is further configured to perform focused ultrasound at the predefined location during the generation of the second set of images based on the training data. Advantageously, a non-invasive and non-radiative means for targeting tissue, e.g., for high intensity deeper tissue treatment, is facilitated.

Preferably, the at least one imaging device is configured to transmit the second set of images to the at least one training device, preferably wirelessly.

Preferably, the at least one training device is configured to update the training data based on the second set of images and further to transmit the updated training data to the at least one imaging device, preferably wirelessly.

Preferably, the at least one training device is configured to control at least one parameter of the at least one imaging device, preferably wirelessly.

Preferably, the at least one parameter comprises the operating frequency of the at least one imaging device and/or focus of the second set of images corresponding to the predefined location and/or depth of the second set of images corresponding to the predefined location.

Advantageously, the reliability of the at least one imaging device can be further improved.

Preferably, the imaging system further comprises a storage, preferably a cloud storage, configured to store the first set of images and/or the second set of images and/or the training data.

According to a second aspect of the invention, an imaging device for medical imaging is provided. The imaging device comprises a flexible substrate configured to be attached to the skin of a person at a predefined location. The imaging device further comprises a processor configured to process training data corresponding to the predefined location based on a first set of images generated in combination with at least one training device according to the first aspect of the invention.

Moreover, the imaging device comprises at least one imaging module configured to generate a second set of images corresponding to the predefined location based on the training data. In this regard, the processor and the at least one imaging module are arranged on the flexible substrate.

Preferably, the flexible substrate is configured to be attached to the skin of the person at the predefined location mechanically or via an adhesive.

Preferably, the at least one imaging module is a transducer, preferably an ultrasound transducer.

Preferably, the imaging device further comprises a transceiver configured to receive the training data from the at least one training device, preferably wirelessly. Additionally or alternatively, the transceiver is configured to transmit the second set of images to the at least one training device, preferably wirelessly.

According to a third aspect of the invention, an imaging method for medical imaging is provided. The method comprises a step of generating, by at least one imaging device being attached to the skin of a person at a predefined location, in combination with at least one training device, a first set of images corresponding to the predefined location. The method further comprises a step of generating, by the at least one training device, training data corresponding to the predefined location based on the first set of images.

Moreover, the method comprises a step of generating, exclusively by the at least one imaging device, a second set of images corresponding to the predefined location based on the training data.

It is to be noted that the method according to the third aspect corresponds to the system according to the first aspect and its implementation forms. It is further to be noted that the imaging device according to the second aspect corresponds to the imaging device according to the first aspect and its implementation forms.

Exemplary embodiments of the invention are now further explained with respect to the drawings by way of example only, and not for limitation. In the drawings:
- Fig. 1: shows a first exemplary embodiment of the imaging system according to the first aspect of the invention;
- Fig. 2: shows a second exemplary embodiment of the imaging system according to the first aspect of the invention;
- Fig. 3: shows a third exemplary embodiment of the imaging system according to the first aspect of the invention;
- Fig. 4: shows an exemplary embodiment of the imaging device according to the second aspect of the invention;
- Fig. 5A: shows an exemplary training phase of the imaging system;
- Fig. 5B: shows an exemplary imaging and/or delivery phase of the imaging device; and
- Fig. 6: shows an exemplary embodiment of the method according to the third aspect of the invention.

Reference will now be made in detail to the embodiments of the present invention, examples of which are illustrated in the accompanying drawings. However, the following embodiments of the present invention may be variously modified and the range of the present invention is not limited by the following embodiments.

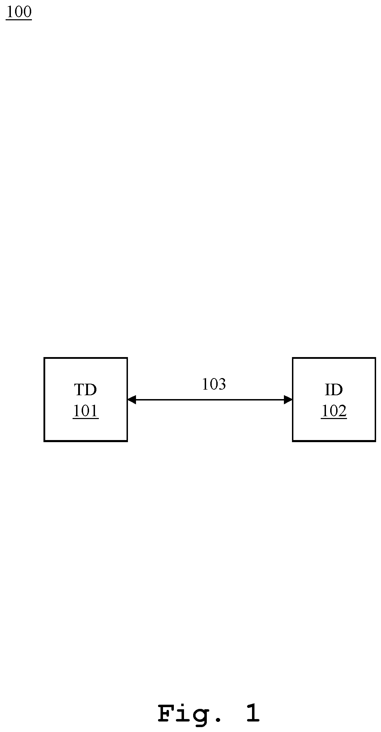

In Fig. 1, a first exemplary embodiment of the imaging system 100 according to the first aspect of the invention is illustrated. In this regard, the term imaging can be understood as acoustic imaging performed at the predefined location, e.g., corresponding to a target organ. Additionally or alternatively, the term imaging can be understood as spatial and/or temporal sensing at the predefined location, e.g., corresponding to a target organ.

The imaging system 100 may comprise a training device (TD) 101 and an imaging or sensing device (ID) 102. The imaging device 102 may be coupled to the training device 101 via a link 103, which can be a wired link or a wireless link. In particular, the imaging device 102 may be attached to the skin of a user, e.g., a patient, at a predefined location, e.g., corresponding to a target organ/tissue.

During a first phase of operation or the training phase, the imaging device 102, in combination with the training device 101, may generate a first set of images and/or sensing data corresponding to the predefined location and/or target organ/tissue, and the training device 101 may generate training data corresponding to the predefined location and/or target organ/tissue based on the first set of images and/or sensing data.

During a second phase of operation or the delivery phase, the imaging device 102 may exclusively generate a second set of images and/or sensing data corresponding to the predefined location and/or target organ/tissue based on the training data and/or may perform a specific task based on the second set of images and/or sensing data, particularly from a location remote to the training device 101.

For example, the training device 101 may comprise an ultrasound imaging device or an x-ray imaging device or a CT device or a MRI device or a PET device or a combination thereof, especially during the training phase to facilitate the training of the imaging device 102.

For example, the images may correspond to ultrasound images of a target organ, e.g., the pancreas, which can be monitored, e.g., for detecting the bubble agent resonance. Additionally or alternatively, the sensing data may comprise unreconstructed/raw sensing data or processed sensing data, from which ultrasound images can be realized, e.g., via the training device 101, of a target organ, e.g., the pancreas. Thus, the images reconstructed from the raw sensing data can also be monitored, e.g., for detecting the bubble agent resonance.

For instance, during the training phase, the training device 101 may generate the training data, which may comprise instructions for the operating frequency of the imaging device 102, e.g., to facilitate focused ultrasound and/or to oscillate the bubble agents at their resonant frequencies. For example, the training device 101 may generate the instructions for the operating frequency of the imaging device 102 for ultrasound imaging of a target organ based on the scan depth of the ultrasound imaging realized from the first set of images and/or sensing data and/or based on the resonance frequency of the bubble agents.

Additionally or alternatively, during the training phase, the training device 101 may generate the training data, which may comprise beamforming information to generate the second set of images and/or sensing data, e.g., the focusing of the beams, corresponding to the predefined location, e.g., to deduce the location/position of a target organ. For example, the training device 101 may generate the beamforming information for the imaging device 102 for ultrasound imaging of the target organ based on the focus of the ultrasound imaging determined from the first set of images and/or sensing data.

Additionally or alternatively, during the training phase, the training device 101 may generate the training data, which may comprise the depth to generate the second set of images and/or sensing data, e.g., the depth of the beams for sensing, corresponding to the predefined location, e.g., corresponding to a target organ/tissue. For example, the training device 101 may generate the instructions for the imaging device 102 for ultrasound imaging of the target organ based on the scan depth of the ultrasound imaging determined from the first set of images and/or sensing data.

Additionally or alternatively, during the training phase, the training device 101 may generate the training data, which may comprise instructions regarding a detection of attachment anomalies, e.g., air bubbles, between the imaging device 102 and the skin, e.g., to generate notifications for the user to reattach/correctly attach the imaging device 102 upon detecting attachment anomalies between the imaging device 102 and the skin.

As such, particularly during the training phase, the training device 101 may train the imaging device 102 by means of the training data, which may comprise the generation of the images of a target organ, e.g., ultrasound images of the pancreas, such that the imaging device 102 may be able to recognize, especially intuitively, the appropriate or correct position of a target organ, e.g., the pancreas, in order to carry out the sensing operation, e.g., focused ultrasound to excite the bubble agents, during the delivery phase.

For instance, the imaging device 102 may transmit the second set of images and/or sensing data to the training device 101 wirelessly, e.g., via the link 103. Accordingly, the training device 101 may update the training data based on the second set of images and/or sensing data and may transmit the updated training data to the imaging device 102 wirelessly, e.g., via the link 103.

Alternatively, the training device 101 may wirelessly control one or more parameters, e.g., the operating frequency, beamforming, depth of sensing, etc., of the imaging device 102, e.g., via the link 103, particularly if a recalibration of the imaging device 102 is needed, e.g., a change in beamforming and/or depth of sensing of the imaging device 102 due to the drift of the imaging device 102 with respect to the target organ caused by a movement, which can be determined based on the second set of images and/or sensing data. For example, the training device 101 may switch on or switch off the imaging device 102 wirelessly, e.g., via the link 103, if needed.

It is to be noted that, during the training phase, the imaging device 102 may generate the first set of images and/or sensing data under the supervision of the training device 101. For example, during the training phase, the training device 101 may comprise a high-resolution ultrasound device and the imaging device 102 may generate ultrasound images of a target organ or raw/processed sensing data in relation to the target organ for ultrasound image reconstruction.

However, during the delivery phase, the imaging device 102 may generate the second set of images and/or sensing data exclusively, e.g., ultrasound images of the target organ, preferably remotely with respect to the training device 101, particularly based on the training carried out during the training phase.

As such, during the training phase, the training device 101, based on the first set of images and/or sensing data, may train the imaging device 102 to recognize when the target organ is in a target location. Accordingly, during the delivery phase, the imaging device 102 may recognize the correct location of the target organ based on the second set of images and/or sensing data, and may proceed with the delivery phase, e.g., focused ultrasound to excite the bubble agents.

In one example, during the delivery phase, the bubble agents may be excited, particularly by the imaging device 102, without human intervention or without any supervision of the imaging device 102. For example, based on the training of the imaging device 102 during the training phase, the imaging device 102 may recognize the correct location of the target organ and may accordingly excite the bubble agents, e.g., by oscillating the bubble agents at their resonant frequency using ultrasound.

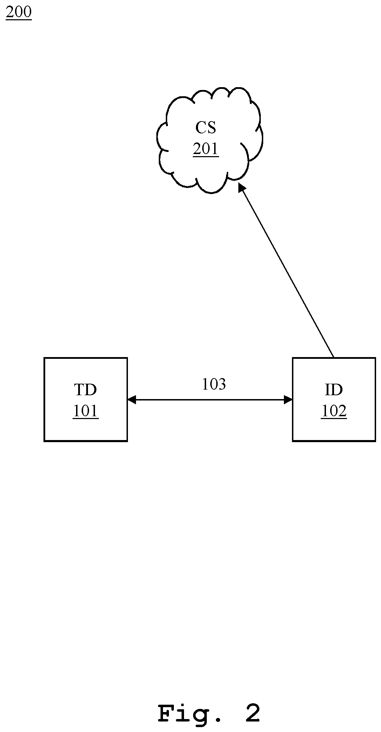

In Fig. 2, a second exemplary embodiment of the imaging system 200 according to the first aspect of the invention is illustrated. The imaging system 200 differs from the imaging system 100 in that the imaging system 200 further comprises a cloud storage (CS) 201. In this regard, the imaging device 102 may be wirelessly coupled to the cloud storage 201.

Accordingly, the imaging device 102 may wirelessly transmit the first set of images and/or sensing data, e.g., during the training phase, the second set of images and/or sensing data, e.g., during the delivery phase, and the training data, e.g., during the training phase and/or delivery phase, to the cloud storage 201 for remote access and/or storage of the data.

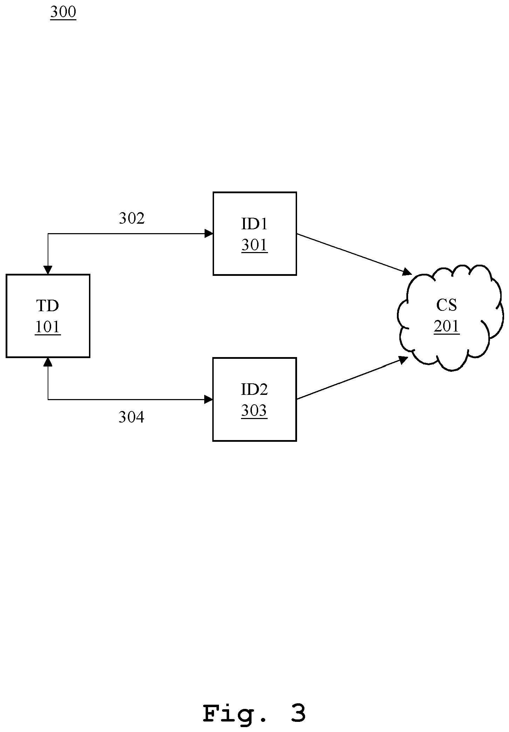

In Fig. 3, a third exemplary embodiment of the imaging system 300 according to the first aspect of the invention is illustrated. The imaging system 300 may comprise the training device 101, a first imaging device 301, and a second imaging device 303, where each of the first imaging device 301 and the second imaging device 303 may correspond to the imaging device 102 of Figs. 1-2. It is to be understood that the imaging system 300 can comprise more than two imaging devices, as depicted herein.

In this regard, the first imaging device 301 may be coupled to the training device 101 via a first link 302, which can be a wired link or a wireless link, and the second imaging device 303 may be coupled to the training device 101 via a second link 304, which can be a wired link or a wireless link.

In particular, the first imaging device 301 may be attached to the skin of a user, e.g., a patient, at a first predefined location, e.g., corresponding to a target organ/tissue, and the second imaging device 303 may be attached to the skin of the user at a second predefined location.

For example, the first imaging device 301 may be attached to the skin of the user to generate abdominal ultrasound images and the second imaging device 303 may be attached to the skin of the user to generate cardiac ultrasound images.

Alternatively, the first imaging device 301 may be attached to the skin of a first user at a first predefined location, and the second imaging device 303 may be attached to the skin of a second user at a second predefined location.

For example, the first imaging device 301 may be attached to the skin of the first user to generate abdominal or cardiac ultrasound images and the second imaging device 303 may be attached to the skin of the second user to generate abdominal or cardiac ultrasound images.

As such, a plurality of imaging devices can be trained by the training device 101, which can be used for targeted drug delivery at the respective plurality of locations or organs or tissue either simultaneously or sequentially.

During the training phase, the first imaging device 301, in combination with the training device 101, may generate images and/or sensing data corresponding to the first predefined location, and the training device 101 may generate training data for the first imaging device 301 corresponding to the first predefined location based on the generated images and/or sensing data.

Sequentially or simultaneously, during the training phase, the second imaging device 303, in combination with the training device 101, may generate images and/or sensing data corresponding to the second predefined location, and the training device 101 may generate training data for the second imaging device 303 corresponding to the second predefined location based on the generated images and/or sensing data.

During the delivery phase, the first imaging device 301 may exclusively generate further images and/or sensing data based on the training data corresponding to the first predefined location, particularly from a location remote to the training device 101.

Sequentially or simultaneously, during the delivery phase, the second imaging device 303 may exclusively generate further images and/or sensing data based on the training data corresponding to the second predefined location, particularly from a location remote to the training device 101.

The imaging system 300 may further comprise the cloud storage 201. In this regard, each of the first imaging device 301 and the second imaging device 303 may be wirelessly coupled to the cloud storage 201.

For instance, the first imaging device 301 may wirelessly transmit the images and/or sensing data, e.g., during the training phase, the images and/or sensing data, e.g., during the delivery phase, and the training data, e.g., during the training phase and/or delivery phase, to the cloud storage 201.

Sequentially or simultaneously, the second imaging device 303 may wirelessly transmit the images and/or sensing data, e.g., during the training phase, the images and/or sensing data, e.g., during the delivery phase, and the training data, e.g., during the training phase and/or delivery phase, to the cloud storage 201.

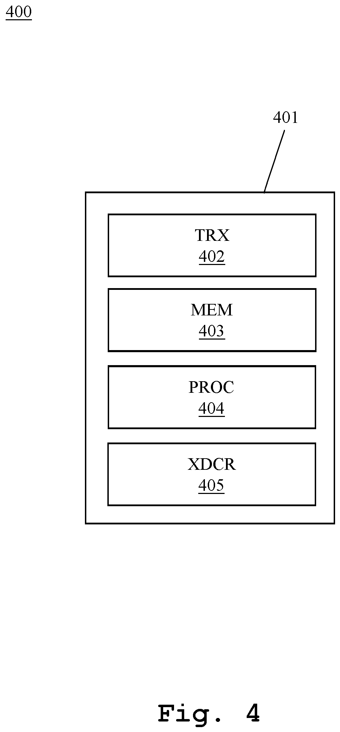

In Fig. 4, an exemplary embodiment of the imaging device 400 according to the second aspect of the invention is illustrated. The imaging device 400 may correspond to the imaging devices 102, 301, 303 of Figs. 1-3.

For example, the imaging device 400 may comprise a flexible substrate 401 that is able to be attached to the skin of a user, e.g., a patient. In this regard, the flexible substrate 401 may be attached to the skin of the user mechanically, e.g., via strap, or via a garment fitted onto the user, or via an adhesive.

For example, the flexible substrate 401 may be a relatively flexible material, e.g., able to elastically deflect over a defined distance without yielding, especially to reduce the risk of drift of the imaging device caused by a movement. The flexible substrate 401 may comprise or be composed of materials including ferro-electric polymers from polyvinylidene fluoride (PVDF) family of materials.

The imaging device 400 may further comprise a transceiver (TRX) 402, especially a wireless transceiver. The transceiver 402 may wirelessly transmit/receive data to/from the training device 101 of Figs. 1-3 and/or the cloud storage of Figs. 2-3.

Additionally or optionally, the imaging device 400 may comprise a local storage or memory (MEM) 403, which may store the training data locally. The memory 403 may further store the images and/or sensing data generated by the imaging device 400.

Additionally or optionally, the imaging device 400 may comprise a processor (PROC) 404, which may process the training data to extract instructions and/or the raw data generated by the imaging device 400.

In addition, the imaging device 400 may comprise an imaging module (XDCR) 405, especially an ultrasound transducer, preferably an array of ultrasound transducers with embedded electronics to facilitate beamforming. In this regard, the processor 404 may control the frequency and/or the beamforming of the imaging module 405 based on the generated images/sensing data and/or the training data.

Although not explicitly shown, the imaging device 400 may comprise a switch, especially a wireless switch, so that the training device 101 may switch on/off the imaging device 400 wirelessly if needed.

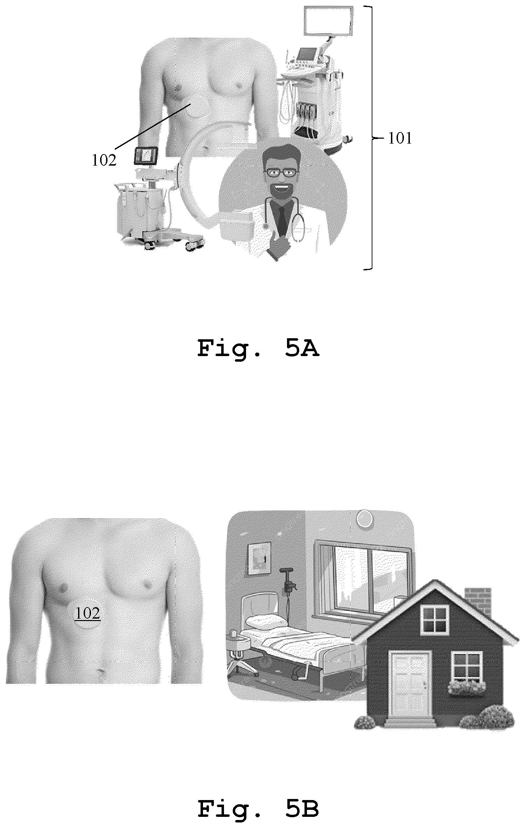

Along Fig. 5A and Fig. 5B, an exemplary operation of the imaging system 100, 200, 300 is illustrated. In particular, Fig. 5A shows an exemplary training phase of the imaging system 100, 200, 300 and Fig. 5B shows an exemplary delivery phase of the imaging system 100, 200, 300.

For example, during the training phase, images and/or sensing data may be generated by one or more imaging devices, e.g., by the imaging device 102, especially in combination with the training device 101. Accordingly, the training device 101 may generate the training data, e.g., for the imaging device 102, based on the generated images and/or sensing data. Preferably, during the training phase, the generation of the images/sensing data and the generation of the training data may be controlled by skilled personnel, e.g., a clinician.

For example, during the delivery phase, images and/or sensing data may be exclusively generated by one or more imaging devices, e.g., by the imaging device 102, especially independent of the training device 101, particularly based on the training data.

For example, the training data may be further updated based on the images and/or sensing data that are generated during the delivery phase, optionally under the control of the skilled personnel, e.g., via accessing the data from the cloud storage 201.

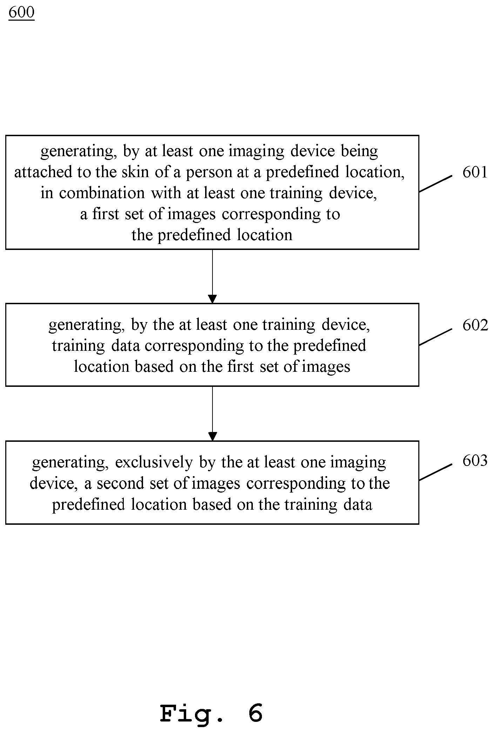

In Fig. 6, an exemplary embodiment of the method 600 according to the third aspect of the invention is illustrated. In a first step 601, a first set of images corresponding to a predefined location are generated by at least one imaging device being attached to the skin of a person at the predefined location in combination with at least one training device.

In a second step 602, training data corresponding to the predefined location are generated by the at least one training device based on the first set of images. In a third step 603, a second set of images corresponding to the predefined location are generated exclusively by the at least one imaging device based on the training data.

It is important to note that, in the description as well as in the claims, the word "comprising" does not exclude other elements or steps and the indefinite article "a" or "an" does not exclude a plurality. A single element or other unit may fulfill the functions of several entities or items recited in the claims.

It should be understood that the term "and/or" used in the specification and the appended claims of this application refers to any combination and all possible combinations of one or more associated listed items, and includes these combinations.

It should also be understood that the word "coupled" implies that the elements may be directly connected together or may be coupled through one or more intervening elements. Moreover, the disclosure with regard to any of the aspects is also relevant with regard to the other aspects of the disclosure.

Although the invention has been illustrated and described with respect to one or more implementations, equivalent alterations and modifications will occur to others skilled in the art upon the reading and understanding of this specification and the annexed drawings. In addition, while a particular feature of the invention may have been disclosed with respect to only one of several implementations, such feature may be combined with one or more other features of the other implementations as may be desired and advantageous for any given or particular application.

## Claims

1. An imaging system (100, 200, 300) for medical imaging comprising:
at least one training device (101), and
at least one imaging device (102, 301, 303) operably coupled to the at least one training device (101), the at least one imaging device (102, 301, 303) being configured to be attached to the skin of a person at a predefined location,
wherein the at least one imaging device (102, 301, 303), in combination with the at least one training device (101), is configured to generate a first set of images corresponding to the predefined location, whereby the at least one training device (101) is configured to generate training data corresponding to the predefined location based on the first set of images, and
wherein the at least one imaging device (102, 301, 303) is further configured to exclusively generate a second set of images corresponding to the predefined location based on the training data.

2. The imaging system according to claim 1,
wherein the at least one training device (101) comprises an ultrasound imaging device or an x-ray imaging device or a computed tomography, CT, device or a magnetic resonance imaging, MRI, device or a positron emission tomography, PET, device or a combination thereof.

3. The imaging system according to claim 1 or 2,
wherein the first set of images and the second set of images are ultrasound images, preferably Doppler ultrasound images.

4. The imaging system according to any of claims 1 to 3, wherein the training data comprises instructions for the operating frequency of the at least one imaging device (102, 301, 303) and/or focus of the second set of images corresponding to the predefined location and/or depth of the second set of images corresponding to the predefined location and/or attachment anomalies between the at least one imaging device (102, 301, 303) and the skin.

5. The imaging system according to any of claims 1 to 4, wherein the training data further comprises parameters for focused ultrasound based on the first set of images and the at least one imaging device (102, 301, 303) is further configured to perform focused ultrasound at the predefined location during the generation of the second set of images based on the training data.

6. The imaging system according to any of claims 1 to 5, wherein the at least one imaging device (102, 301, 303) is configured to transmit the second set of images to the at least one training device (101), preferably wirelessly.

7. The imaging system according to claim 6,
wherein the at least one training device (101) is configured to update the training data based on the second set of images and further to transmit the updated training data to the at least one imaging device (102, 301, 303), preferably wirelessly.

8. The imaging system according to any of claims 1 to 7,
wherein the at least one training device (101) is configured to control at least one parameter of the at least one imaging device (102, 301, 303), preferably wirelessly.

9. The imaging system according to claim 8,
wherein the at least one parameter comprises the operating frequency of the at least one imaging device (102, 301, 303) and/or focus of the second set of images corresponding to the predefined location and/or depth of the second set of images corresponding to the predefined location.

10. The imaging system according to any of claims 1 to 9, wherein the imaging system further comprises a storage (201), preferably a cloud storage, configured to store the first set of images and/or the second set of images and/or the training data.

11. An imaging device (400) for medical imaging comprising:
a flexible substrate (401) configured to be attached to the skin of a person at a predefined location,
a processor (404) configured to process training data corresponding to the predefined location based on a first set of images generated in combination with at least one training device (101) according to any of claims 1 to 10, and
at least one imaging module (405) configured to generate a second set of images corresponding to the predefined location based on the training data,
wherein the processor (404) and the at least one imaging module (405) are arranged on the flexible substrate (401).

12. The imaging device according to claim 11,
wherein the flexible substrate (401) is configured to be attached to the skin of the person at the predefined location mechanically or via an adhesive.

13. The imaging device according to claim 11 or 12, wherein the at least one imaging module (405) is a transducer, preferably an ultrasound transducer.

14. The imaging device according to any of claims 11 to 13, wherein the imaging device further comprises a transceiver (402) configured to:
receive the training data from the at least one training device (101), preferably wirelessly, and/or
transmit the second set of images to the at least one training device (101), preferably wirelessly.

15. An imaging method (600) for medical imaging comprising:
generating (601), by at least one imaging device being attached to the skin of a person at a predefined location, in combination with at least one training device, a first set of images corresponding to the predefined location,
generating (602), by the at least one training device, training data corresponding to the predefined location based on the first set of images, and
generating (603), exclusively by the at least one imaging device, a second set of images corresponding to the predefined location based on the training data.
